# EUROPÄISCHE PATENTSCHRIFT

(11) **EP 0 464 582 B1**
(45) Veröffentlichungstag und Bekanntmachung des Hinweises auf die Patenterteilung: **01.05.1996**
(21) Anmeldenummer: 91110432.1
(22) Anmeldetag: 25.06.1991
(51) Int. Cl.: C07D 475/14

(54) **Verfahren zur Reinigung von fermentativ hergestelltem Riboflavin**
Process for the purification of riboflavin obtained by fermentation
Procédé pour la purification de riboflavine obtenu par fermentation

(30) Priorität: 04.07.1990 DE 4021274
(43) Veröffentlichungstag der Anmeldung: 08.01.1992
(73) Patentinhaber: BASF Aktiengesellschaft, D-67063 Ludwigshafen (DE)
(72) Erfinder: Grimmer, Johannes, W-6700 Ludwigshafen (DE); Kiefer, Hans, Dr., W-6706 Wachenheim (DE); Martin, Christoph, Dr., W-6800 Mannheim 1 (DE)

(56) Entgegenhaltungen:
- EP-A- 0 112 538
- EP-A- 0 137 226
- EP-A- 0 164 704
- EP-A- 0 211 289
- US-A- 2 822 361
- US-A- 4 165 250
- US-A- 4 165 250
- Vitamine, Chemie und Biochemie, Band II, Dr. J. Fragner, Prag, Gustav Fischer Verlag Jena, 1965.

## Beschreibung

Die Erfindung betrifft ein Verfahren zur Reinigung von auf mikrobiologischem Wege hergestelltem Riboflavin (Vitamin B2) durch Erhitzen des verunreinigten Riboflavins in Gegenwart von Wasser oder wäßrigen Säuren.

Die Herstellung von Riboflavin durch mikrobielle Fermentationsprozesse ist bekannt. So kann Riboflavin z.B. gemäß DE 29 20 592 durch Fermentation von Ashbya gossypii oder Eremothecium ashbyii, gemäß EP 211 289 durch Fermentation von zu Saccharomyces oder einer Variante davon gehörender Hefe, gemäß EP 231 605 mittels Candida flareri und gemäß DE 34 20 310 mittels Bacillus subtilis gewonnen werden. Das auf diesem Wege technisch hergestellte Riboflavin dient im allgemeinen als Tierfuttermittelzusatz. Das Endprodukt kann bei diesen Verfahren einfach durch Eindampfen der erhaltenen Kulturflüssigkeit zusammen mit der noch enthaltenen Biomasse in Form eines Riboflavinkonzentrats erhalten werden. Hierbei erhält man ein Produkt, das je nach Leistungsfähigkeit des Riboflavin produzierenden Stammes 20 bis 40 Gew.-% Riboflavin enthält.

Ein Produkt mit einem Riboflavingehalt von etwa 40 bis 60 Gew.-% erhält man beispielsweise dadurch, daß man die Riboflavin enthaltende Kulturbrühe auf spezielle Weise ein- oder mehrfach dekantiert (vgl. DE-A 29 20 592).

So wird auf fermentativem Wege hergestelltes Riboflavin in verschiedenen Qualitäten als Futtermittelzusatz kommerziell angeboten. Die feed grade Marktprodukte weisen im allgemeinen Riboflavingehalte von 50 bis etwa 96 Gew.-% auf. Insbesondere Produkte mit Gehalten von etwa 65 %, etwa 80 % oder etwa 96 Gew.-% Riboflavin werden angeboten.

Die so erhaltenen Produkte erfüllen jedoch nicht die Anforderungen an Riboflavin mit Pharmaqualität. Beispielsweise schreiben die Pharmakopoen hierfür einen Mindestgehalt von 98 Gew.-% an Riboflavin vor.

Relativ reines Riboflavin erhält man gemäß dem Verfahren von EP 137 226 durch Trennen des in der speziellen Kultur enthaltenen Riboflavins in Form einer erwärmten wäßrigen Lösung von festen Bestandteilen und Kristallisieren des Riboflavins aus dieser erwärmten Lösung. Nachteilig an diesem Verfahren ist, daß man enorm große Mengen Wasser einsetzen muß, um das in Wasser schwer lösliche Riboflavin in Lösung zu bringen. Für einen Einsatz in technischem Maßstab ist das Verfahren daher ungeeignet.

Es war daher die Aufgabe der Erfindung, ein Reinigungsverfahren für durch mikrobielle Fermentation hergestelltes Riboflavin zur Verfügung zu stellen, das es erlaubt, auch aus fermentativ hergestelltem Riboflavin ein Produkt mit Pharmaqualität bzw. Lebensmittelqualität zu gewinnen. Ganz allgemein war es auch die Aufgabe der Erfindung, ein Verfahren zu entwickeln, mit dessen Hilfe es auf einfache Weise gelingt, den Riboflavingehalt von fermentativ hergestelltem Riboflavin wesentlich zu erhöhen.

Gegenstand der Erfindung ist daher ein Verfahren zur Reinigung von fermentativ hergestelltem Riboflavin, das dadurch gekennzeichnet ist, daß man das verunreinigte Riboflavin in Wasser oder in verdünnter wäßriger Säure suspendiert und die Suspension unter Rühren 0,3 bis 3 Stunden, vorzugsweise 1 bis 2,5 Stunden, auf Temperaturen von 75 bis 130°C, vorzugsweise 80 bis 120°C erhitzt und nach dem Abkühlen des Reaktionsgemisches die gebildeten Kristalle in an sich bekannter Weise isoliert.

Bei diesem Erhitzen in Wasser oder unverdünnter wäßriger Säure tritt vermutlich eine Kristallumwandlung auf, was sich dadurch bemerkbar macht, daß die Kristallmasse zäh und breiig wird. Ein Rühren der Masse ist daher unumgänglich. Bei weiterem Erhitzen bildet sich wieder eine normale Suspension, aus der das unter Kristallumwandlung gereinigte Riboflavin in an sich bekannter Weise isoliert werden kann.

Das Wasser wendet man hierbei in der etwa 10- bis 30-fachen, vorzugsweise 15- bis 20-fachen Gewichtsmenge, bezogen auf Riboflavin an.

Durch das erfindungsgemäße Erhitzen mit Wasser kann beispielsweise etwa 96 gew.-%iges Riboflavin in etwa 99 gew.-%iges, etwa 90 gew.-%iges in etwa 97 gew.-%iges und etwa 65 gew.-%iges in etwa 80 gew.-%iges Riboflavin überführt werden.

Der Reinigungseffekt kann noch dadurch verbessert werden, daß man dem Wasser 0,05 bis 10 Gew.-%, vorzugsweise 0,5 bis 3 Gew.-% einer in Wasser löslichen Säure, vorzugsweise eine Mineralsäure wie H₂SO₄, H₃PO₄ oder HCl zumischt.

Durch entsprechendes Erhitzen des verunreinigten Vitamins B2 in verdünnten wäßrigen Säuren kann man beispielsweise aus etwa 96 gew.-%igem Riboflavin 100 gew.-%iges, aus etwa 90 gew.-%igem Riboflavin etwa 97 gew.-%iges und aus etwa 65 gew.-%igem Riboflavin etwa 90 gew.-%iges Riboflavin erhalten.

Unter der Bezeichnung verdünnte wäßrige Säure verstehen wir im Rahmen dieser Erfindung die Lösung einer in Wasser löslichen Säure, vorzugsweise einer Mineralsäure, die das Riboflavinmolekül nicht beeinträchtigt. Als in Wasser lösliche Säuren seien beispielsweise genannt organische Säure, wie Ameisensäure, Weinsäure, Citronensäure oder Essigsäure und Mineralsäure, wie Schwefelsäure, Salzsäure oder Phosphorsäure.

Die Natur der Säure ist ohne Belang, solange sie völlig in Wasser löslich ist, das Riboflavinmolekül nicht angreift und die Konzentration der Säure nicht so hoch ist, daß Riboflavin gelöst wird.

Mit besonderem Vorteil arbeitet man mit den gängigen Mineralsäuren, wie H₄SO₄, H₃PO₄ oder HCl.

Bei Verwendung von verdünnter Schwefelsäure hat sich der Einsatz einer etwa 0,1 bis 1 molaren Schwefelsäure als besonders vorteilhaft erwiesen, bei Verwendung von verdünnter Phosphorsäure oder Salzsäure der Einsatz einer etwa 0,1 bis 1,5 molaren Säure.

Mit Hilfe des erfindungsgemäßen Verfahrens kann fermentativ hergestelltes Riboflavin auf technisch einfache Weise gereinigt werden.

Die folgenden Beispiele sollen das erfindungsgemäße Verfahren veranschaulichen. Die in den Beispielen angegebenen Riboflavin-Gehalte wurden in allen Fällen gemäß der Pharmakopoe Europe II bestimmt.

### Beispiel 1

100 g eines handelsüblichen Riboflavins mit einem Gehalt von 95,7 Gew.-% wurden unter Rühren in 2000 ml destilliertes Wasser eingetragen. Die gut rührbare Suspension wurde im Wasserbad auf 80°C erwärmt, wonach innerhalb von 30 Minuten (min) eine Kristallumwandlung erfolgte. Die gebildete zähe und breiige Kristallmasse wurde beim weiteren Erhitzen auf 100°C wieder zu einer gut rührbaren Suspension. Nach etwa einer Stunde (h) Verweilzeit bei 100°C wurde auf 30°C abgekühlt und abfiltriert. Der Filterkuchen wurde mit 1000 ml eines 40°C warmen Wasser und anschließend mit 1000 ml Methanol gewaschen. Nach dem Trocknen unter vermindertem Druck bei 80°C betrug die Ausbeute 95,32 g und die Reinheit 98,9 %.

### Beispiel 2

25 g eines handelsüblichen Riboflavins mit einem Gehalt von 95,7 Gew.-% wurde in eine Mischung aus 490 ml vollentsalztem Wasser und 10 ml konz. H₂SO₄ eingetragen. Die erhaltene Suspension wurde zum Sieden erhitzt und bei 95 bis 97°C 1 h lang gut durchgeführt. Anschließend wurde auf 40°C abgekühlt, von der Mutterlauge abgesaugt und der Filterkuchen mit 500 ml eines 40°C warmen Wassers und dann mit 500 ml Methanol gewaschen. Nach dem Trocknen unter vermindertem Druck bei 80°C betrug die Ausbeute 23,49 g und die Reinheit 99,95 %.

### Beispiel 3

In einer für überdruck zugelassenen Rührapparatur wurden 6000 ml vollentsalztes Wasser und 50 g einer 85 %igen Phsophorsäure vorgelegt und dann 1532 g eines noch feuchten Vitamins B2 (Trockensubstanz 23 %, entsprechend 352,36 g Vitamin B2 trocken mit einem Gehalt von 90 % Vitamin B2) eingetragen.

Die Mischung wurde in der geschlossenen Rührapparatur auf 120°C erhitzt und bei dieser Temperatur 1 h lang gerührt. Der sich einstellende Druck betrug 1,1 bis 1,3 bar. Nach 1 h wurde auf 40°C abgekühlt, von der Mutterlauge abgesaugt und der Filterkuchen zunächst mit 3000 ml vollentsalztem Wasser und dann mit 2000 ml Methanol gewaschen. Nach Trocknen unter vermindertem Druck bei 80°C betrug die Ausbeute 318 g und die Reinheit 96,8 %.

### Beispiel 4

Es wurde gearbeitet wie in Beispiel 3, jedoch ohne Zusatz von Phosphorsäure.

Es wurden 320 g Vitamin B2 mit einer Reinheit von 95,62 % erhalten.

### Beispiel 5

In einer für überdruck zugelassenen Rührapparatur wurden 8000 ml vollentsalztes Wasser vorgelegt und hierzu unter Rühren 400 g eines handelsüblichen feed grade Vitamins B2 mit einem Gehalt von 65 % Vitamin B2 addiert. Die erhaltene Suspension wurde in der geschlossenen Apparatur auf 120°C erhitzt und bei dieser Temperatur 1 h gerührt. Anschließend wurde auf 40°C abgekühlt, von der Mutterlauge abgesaugt und der Filterkuchen zunächst mit 4000 ml Wasser und dann mit ca. 2000 ml Methanol gewaschen. Nach Trocknen unter vermindertem Druck bei 80 bis 90°C betrug die Ausbeute 315 g und die Reinheit 79,4 %.

### Beispiel 6

In der in Beispiel 5 beschriebenen Apparatur wurde eine Mischung aus 8000 ml vollentsalztem Wasser und 60 ml einer 85 %igen Phosphorsäure mit 400 g eines handelsüblichen Vitamins B2 mit einem Gehalt von 65 % Vitamin B2 versetzt und die erhaltene Suspension 1 h bei 120°C gerührt. Aufarbeitung analog Beispiel 5 ergab 293,8 g Vitamin B2 einer Reinheit von 90,5 %.

## Patentansprüche

1. Verfahren zur Reinigung von fermentativ hergestelltem und in an sich bekannter Weise isoliertem und ggf. vorgereinigtem Riboflavin, dadurch gekennzeichnet, daß man das verunreinigte Riboflavin in der etwa 10- bis 30-fachen Gewichtsmenge Wasser oder verdünnter wäßriger Säure suspendiert und die Suspension unter Rühren 0,3 bis 3 Stunden auf Temperaturen von 75 bis 130°C erhitzt und nach dem Abkühlen die gebildeten Kristalle isoliert.

2. Verfahren Gemäß Anspruch 1, dadurch gekennzeichnet, daß man das verunreinigte Riboflavin in Wasser suspendiert und die Suspension auf Temperaturen von 80 bis 120°C erhitzt.

3. Verfahren gemäß Anspruch 1, dadurch gekennzeichnet, daß man das verunreinigte Riboflavin in verdünnter wäßriger Mineralsäure suspendiert und die Suspension auf Temperaturen von 80 bis 120°C erhitzt.

4. Verfahren gemäß Anspruch 1, dadurch gekennzeichnet, daß man als verdünnte wäßrige Säure etwa 0,1 bis 1 molare Schwefelsäure verwendet.

5. Verfahren gemäß Anspruch 1, dadurch gekennzeichnet, daß man als verdünnte wäßrige Säure etwa 0,1 bis 1,5 molare Phosphorsäure oder Salzsäure verwendet.

6. Verfahren gemäß Anspruch 1, dadurch gekennzeichnet, daß man die Suspension 1 bis 2,5 Stunden auf Temperaturen von 80 bis 120°C erhitzt.

7. Verfahren gemäß Anspruch 1, dadurch gekennzeichnet, daß man das verunreinigte Riboflavin in der etwa 15- bis 20-fachen Gewichtsmenge an Wasser oder verdünnter wäßriger Säure suspendiert.

## Claims

1. A process for purifying riboflavin which has been produced by fermentation and isolated and, where appropriate, prepurified in a conventional way, which comprises suspending the impure riboflavin in about 10 to 30 times the weight of water or dilute aqueous acid, and heating the suspension while stirring at from 75 to 130°C for from 0.3 to 3 hours and, after cooling, isolating the crystals which have formed.

2. A process as claimed in claim 1, wherein the impure riboflavin is suspended in water, and the suspension is heated to from 80 to 120°C.

3. A process as claimed in claim 1, wherein the impure riboflavin is suspended in dilute aqueous mineral acid, and the suspension is heated to from 80 to 120°C.

4. A process as claimed in claim 1, wherein about 0.1-1 molar sulfuric acid is used as dilute aqueous acid.

5. A process as claimed in claim 1, wherein about 0.1-1.5 molar phosphoric acid or hydrochloric acid is used as dilute aqueous acid.

6. A process as claimed in claim 1, wherein the suspension is heated at from 80 to 120°C for from 1 to 2.5 hours.

7. A process as claimed in claim 1, wherein the impure riboflavin is suspended in about 15 to 20 times the weight of water or dilute aqueous acid.

## Revendications

1. Procédé pour purifier la riboflavine préparée par fermentation, isolée de manière connue en soi et le cas échéant soumise à une purification préalable, caractérisé en ce que l'on met la riboflavine impure en suspension dans environ 10 à 30 fois son poids d'eau ou d'un acide aqueux dilué et on chauffe la suspension sous agitation pendant 0,3 à 3 h à des températures de 75 à 130°C puis, après refroidissement, on isole les cristaux formés.

2. Procédé selon la revendication 1, caractérisé en ce que l'on met la riboflavine impure en suspension dans l'eau et on chauffe la suspension à des températures de 80 à 120°C.

3. Procédé selon la revendication 1, caractérisé en ce que l'on met la riboflavine impure en suspension dans un acide minéral aqueux dilué et on chauffe la suspension à des températures de 80 à 120°C.

4. Procédé selon la revendication 1, caractérisé en ce que l'on utilise, en tant qu'acide aqueux dilué, de l'acide sulfurique environ 0,1 à 1 M.

5. Procédé selon la revendication 1, caractérisé en ce que l'on utilise, en tant qu'acide aqueux dilué, de l'acide phosphorique ou de l'acide chlorhydrique environ 0,1 à 1,5 M.

6. Procédé selon la revendication 1, caractérisé en ce que l'on chauffe la suspension pendant 1 à 2,5 h à des températures de 80 à 120°C.

7. Procédé selon la revendication 1, caractérisé en ce que l'on met la riboflavine impure en suspension dans environ 15 à 20 fois son poids d'eau ou d'un acide aqueux dilué.
